# EUROPEAN PATENT APPLICATION

(11) **EP 1 990 010 A1**
(43) Date of publication of application: **12.11.2008**
(21) Application number: 08008458.5
(22) Date of filing: 05.05.2008
(51) Int. Cl.: A61B 8/00, A61B 8/08, A61B 8/14, G01S 15/89, G01S 7/539, A61N 7/02, G01S 7/52

(54) **Ultrasound imaging system**

(30) Priority: 07.05.2007 JP 2007122549
(71) Applicant: Hitachi, Ltd., Chiyoda-ku Tokyo 100-8280 (JP)
(72) Inventor: Kawabata, Kenichi c/o Hitachi, Ltd., IP Group, Chiyoda-ku Tokyo 100-8220 (JP)
(74) Representative: Strehl Schübel-Hopf & Partner

(57) **Abstract**

The present invention aims to provide a tissue-function measurement device and a therapeutic device which are not dependent on tissue shape. According to the present invention, the stiffness of a tissue is measured by using, as an index, acoustic variation during the generation of microbubbles from phase-change nano droplets. The acoustic variation is, for example, a duration time of the generation of a harmonic.

## Description

### CLAIM OF PRIORITY

The present application claims priority from Japanese application JP 2007-122549 filed on May 7, 2007, the content of which is hereby incorporated by reference into this application.

### BACKGROUND OF THE INVENTION

### 1. Field of the Invention

The present invention relates to an ultrasound imaging system for diagnosis and therapy which is used in combination with a phase-change nano droplet ultrasound contrast agent.

### 2. Description of the Related Art

It has been a long time since diagnostic image modalities, such as X-ray computerized tomography (CT), magnetic resonance imaging (MRI), and ultrasound diagnostic equipment, became indispensable tools in the medical field. These modalities images each of differences in CT value, spin relaxation time, and acoustic impedance in a biological body. Since such differences in physical properties mainly reflect a structure (shape) of a biological body, the imaging thereof is called "morphological imaging." In contrast, an image modality for imaging certain sites which are the same in terms of structure but different in terms of functionality is called "functional imaging." One type of the functional imaging which is specifically configured to visualize molecular biological information, that is, the state of biological components, such as proteins, amino acids, and nucleic acids, is called "molecular imaging." Highly expected to be applied to the elucidation of life phenomena, such as development and differentiation, and to diagnosis and therapy of diseases, molecular imaging is currently one of the research fields attracting the most attention. In molecular imaging, it is common to utilize a "molecular probe" which is a substance having a structure with a selectivity towards a biological component. In the case of molecular imaging utilizing a molecular probe, the molecular probe is added with a structure which allows the detection of the molecular probe by use of a certain physical means, and thereby the in vivo distribution of the molecular probe is noninvasively visualized from the outside of the body. In a report published in Nature Rev. Cancer 2: 750-763 (2002) by Allen, an example of a molecular probe targeting a tumor is described. Peptides, antigens, and the like are commonly used as molecular probes.

A positron emission tomography (PET) system and an optical imaging system are virtually specialized in performing molecular imaging. The former of these systems has been widely used as a clinical tool for classifying the degree of tumor spread and the progression (stage) of a tumor, while the latter has been widely used as a noninvasive pharmacokinetic analytical tool using laboratory animals in the fields of drug development and the like.

In addition to these systems specialized in performing molecular imaging, new systems capable of detecting and diagnosing diseases earlier than a conventional system have been under development on the basis of existing modalities used for morphological imaging, such as MRI and ultrasound. Among such new systems, a system using ultrasound has the following unique features which are not given to other modalities: (1) being capable of performing superior real-time detection; (2) having a small size, thereby involving less restriction in the use in an operation room; and (3) being usable as a tool for diagnosis as well as for therapy. Therefore, it is expected that such an ultrasound-based system be an integrated tool for both diagnosis and therapy which can be used in not only large hospitals but also other smaller hospitals.

Ultrasound used as a therapeutic tool is, in principle, capable of providing high energy required for therapy only to an affected area by irradiating the affected area with a focused ultrasound away from the affected area, especially from the outside of a body, while focusing on the affected area. Therefore, ultrasound has a potential to be an extremely low invasive therapeutic method. A therapeutic method using ultrasound which has been attracting increasing attention in recent years is a thermal coagulation therapy in which a temperature at a target site is increased to a protein denaturation temperature (approximately 65°C) or above in a short period of time as short as a few seconds. As utilizing high intensity focused ultrasound (HIFU) having an intensity of 1 kW/cm² or above, the thermal coagulation therapy is often referred to as HIFU treatment. Currently, HIFU treatment has been drawing attention as a low-invasive therapeutic method for the treatment of breast cancer and prostatic hypertrophy. In HIFU treatment, the site selectivity solely depends on the convergence of ultrasound energy. Consequently, there is a risk of causing severe adverse effects because a site other than the target site may be irradiated by mistake with the high intensity ultrasound having an intensity of 1 kW/cm² or above if body movement or the like causes misalignment in the focus of the ultrasound on the target site.

For this reason, in order to provide highly safe therapy, it is desirable to use a therapeutic method having a different site selectivity in addition to the site selectivity that solely depends on the convergence of ultrasound energy. Use of an agent has been under consideration to achieve a different selectivity in addition to the selectivity depending on such ultrasound. In such a situation, expectations have been raised for microbubbles which are often employed as an ultrasound contrast agent. This is because, as described in a report published in IEEE Trans, UFFC 52: 1690-1698 (2005) by Umemura et al. (hereinafter referred to as non-patent document 2), it has been learned that the presence of microbubbles in a site increases an apparent ultrasound absorption coefficient of the site, and therefore a temperature increase at the site where the microbubbles are located is larger than that at a site with no microbubbles, even if these two sites are irradiated with ultrasound having the same energy level. Hence, if microbubbles can be localized only at a target site, it is possible to selectively heat only the target site by use of this phenomenon of temperature raise. However, it is difficult to localize microbubbles in a tissue, such as a tumor, since the size of microbubbles only allows them to be located in blood vessels.

In addition, there is another biological action of ultrasound involving microbubbles, which is an action due to (acoustic) cavitation. Cavitation is originally a phenomenon in which ultrasound causes bubble nucleation, and in which bubbles grow from the bubble nuclei and then have compression failure. Having microbubbles present in a system is equivalent to being in the stage in which bubbles have grown in the process of cavitation. Accordingly, ultrasound irradiation under the presence of microbubbles allows one of the procedures required for the generation of cavitation to be omitted. Thus, it is known that such ultrasound irradiation under the presence of microbubbles requires only a lower acoustic intensity for the generation of cavitation than otherwise. Once cavitation is generated, the temperature and the pressure reach several thousand degrees Celsius and several hundred atmospheres, respectively, at the last stage where the bubbles collapse. It is known that such high temperature and high pressure cause biological effects either directly or indirectly through a chemical substance called an acoustic cavitation promoting substance as described in Japanese Patent No.3565758. More specifically, such biological effects are known to be cell death and tissue destruction at a site where cavitation is generated.

In the meantime, it has been examined to use an agent such as the one described in Jpn. J. Appl. Phys. 44: 4548-4552 (2005) by Kawabata et al. (hereinafter referred to as non-patent document 3), as a contrast agent. The agent is in form of nanosize droplets when administered to a biological body, and changes its phase upon receiving ultrasound irradiation, resulting in the generation of microbubbles. Being in form of nanosize droplets, the agent can enter a tissue, such as a tumor. Furthermore, a tissue selectivity can be conferred to the agent by using the above-described molecular imaging technique for adding a molecular probe. Application of such a phase-change contrast agent enables ultrasound imaging to be performed in a highly tissue-selective manner.

The above-described functional imaging (molecular imaging) is to image molecular biological characteristics of diseases to provide information different from that provided by morphological imaging. Besides this functional imaging, there is another imaging called tissue functional imaging for visualizing characteristics of a tissue other than morphological characteristics. Tissue functional imaging of particular interest is elasticity imaging which visualizes variation in stiffness within a tissue. By use of a diagnostic device, this imaging aims to obtain information which is obtained by palpation performed by doctors. As proven by the fact that detection of a lump leads to early discovery of breast cancer, stiffness is an important factor reflecting tissue characteristics, such as canceration. If stiffness at a micro area can be visualized by a diagnostic device, it is possible to diagnose pathological conditions, such as arteriosclerosis, which cannot be detected by palpation.

Stiffness detected by palpation is expressed as shear modulus (modulus of elasticity in shear). In a method commonly used for imaging of modulus of elasticity, an operator presses a probe onto the body surface of a patient to obtain a local deformation ratio (distortion) in a body tissue so that distribution of stiffness can be obtained. It is possible to fully visualize a disease area by acquiring the relative modulus of elasticity instead of an absolute value of the modulus of elasticity, on the grounds that modulus of elasticity in shear is one of the physical values which are difficult to accurately measure, and that elasticity imaging can demonstrate its strength in detection of early-stage diseases which are difficult to accurately identify in conventional diagnostic images. For these reasons, it is common to perform diagnosis based on relative modulus of elasticity in clinical practice.

### SUMMARY OF THE INVENTION

By the practical application of functional (molecular) imaging and tissue functional imaging described above, which are capable of complementing information to conventional morphological imaging, it is possible to check the status of diseases, such as cancers, for which early detection is critical, by use of diagnostic imaging. Even in the case where a cancer is discovered in its early stage, conventional therapeutic procedures, such as surgical procedures, radiation therapy, and antineoplastic drug therapy are difficult to apply since these procedures impose a great burden on a patient. To cope with this problem, new therapeutic methods which are less invasive than the conventional therapeutic methods have been developed. In such low-invasive therapeutic methods, a target site to be treated is focused not visually but by use of a diagnostic imaging device; thus, the above-described functional imaging and tissue functional imaging have come to gain significance.

An affected area is distinguished from a normal tissue by a selectivity of an agent in functional (molecular) imaging. However, it is common that not all of an agent administered to a body accumulates in a disease site, and, in fact, part of the agent is distributed in normal tissues surrounding a disease site; thus, it is not possible to strictly distinguish the disease site from the normal tissues. Likewise, the elasticity imaging, which is tissue functional imaging, can visualize a hard region, such as a tumor, but has a difficulty in strictly distinguishing the border between a disease site and normal tissues.

As described above, there is no method available for strictly distinguishing a disease area from a normal area, especially for the purpose of focusing a target site to be treated; thus, it is common that a slightly larger focus area is set for an area to be treated in actual medical treatment. In such treatment, a part of normal tissue is inevitably subjected to the treatment. Thus, it is necessary to develop a method for distinguishing between a disease area and a normal tissue area in order to improve the safety of the treatment. Hence, the present invention aims to provide a tissue function measurement method, a diagnostic device, and a therapeutic device, which are capable of distinguishing between a disease area and a normal tissue area.

The inventors of the present invention have presumed that the combination of molecular imaging and tissue functional imaging performed simultaneously can solve the above-described problem that it is difficult to visualize a border between a disease area and a normal tissue area when a contrast agent is distributed in a normal tissue area in functional (molecular) imaging or when the border has a complicated shape in tissue functional imaging. To be more specific, the problem can be solved by a method for examining whether a site in which a contrast agent is distributed is a disease tissue or a normal tissue by performing elasticity imaging on the basis of a signal from a contrast agent. After having been repeatedly conducting keen investigation according to the presumption, the inventors of the present invention discovered that, when a phase-change nano droplet contrast agent is irradiated with an ultrasound for phase change, the stiffness of a site in which the nano droplets are located causes the variation in: a time required for the generation of microbubbles after the irradiation; the size of a nonlinear component in an acoustic signal generated by the generated microbubbles; and a duration time of the generation of the acoustic signal. This discovery has led the inventors to complete the present invention.

To be more specific, a tissue function measurement device according to the present invention includes: a means for irradiating a phase-change nano droplet ultrasound contrast agent with an ultrasound energy for causing a phase change of the phase-change nano droplet ultrasound contrast agent to generate microbubbles; a means for measuring a time from the phase change of the phase-change nano droplet ultrasonic contrast agent to the generation of microbubbles or for receiving an acoustic signal radiated when the phase-change nano droplet ultrasonic contrast agent are caused to have a phase change and microbubbles are generated; and a means for calculating and recording, and displaying, if necessary, in superimposition with other image information, such as an ultrasound echogram, any one of a time from the phase change of the phase-change nano droplet ultrasonic contrast agent to the generation of microbubbles, the size of a nonlinear component contained in the radiated acoustic signal, and a duration time of the acoustic signal. In the present invention, the phase-change nano droplet ultrasound contrast agent is prepared, as described in the non-patent document 3, by stabilizing a substance having a boiling temperature of body temperature or below and poor water solubility by use of a material having a surfactant effect to obtain droplets having a diameter of a micron or smaller so as to achieve the same condition as being superheated (pseudo superheating). In this pseudo superheated condition, an apparent boiling point of the poorly water-soluble substance is increased; therefore, the substance can remain in form of droplets even when administered to a body and exposed to body temperature or a higher temperature. When the poorly water-soluble substance in this state is given adequate physical stimulation, for example, an ultrasound pulse, the pseudo-superheated condition is released, and then bubbles having a diameter of a few microns are generated by vaporization of the poorly water-soluble substance having its boiling point back to the original. It has been recently revealed that such bubbles are not only a good contrast agent for an ultrasound diagnostic device but also, as shown in the non-patent document 2, a useful sensitizer for therapy using ultrasound as a heating source.

The means for irradiating a phase-change nano droplet ultrasound contrast agent with an ultrasound energy for causing a phase change of the phase-change nano droplet ultrasound contrast agent to generate microbubbles is not especially limited as long as the means is capable of irradiating an area having a size of approximately 2 cm square or smaller in a body with an ultrasound having a frequency in an approximate range from 0.5 MHz to 10 MHz and an intensity (SPTP) in a range from a few W/cm² to several hundred W/cm² for an approximate time of 0.1 µs to 100 ms. The irradiation may be performed with an ultrasound containing multiple frequency components. The means for measuring a time from the phase change of the phase-change nano droplet ultrasonic contrast agent to the generation of microbubbles is not especially limited as long as the means is capable of detecting vaporization of the droplets having a size of several hundred nanometers into a gas having a diameter of a few microns. However, it is desirable to employ any of the following means: a means for optically measuring a difference in scattering intensity between the droplets and the bubbles; a means for measuring a difference in scattering intensity between the droplets and the bubble by use of ultrasound; and a means for detecting a nonlinear component which is rarely generated in the droplets but observed at a significant level in the bubbles among acoustic signals generated in response to the ultrasound irradiated for causing the phase change described above. Although not especially limited, the means for receiving an acoustic signal radiated when the phase-change nano droplet ultrasonic contrast agent is caused to have a phase change and microbubbles are generated is desirable to be configured so as to measure an ultrasound in a frequency bandwidth same as and half or double of that of the ultrasound used for the phase change of the phase-change nano droplet ultrasound contrast agent at a temporal resolution of approximately 0.1 µs or below, in the case where the means is used for the purpose of measuring a chronological change of the acoustic signal. Furthermore, regarding the means for receiving an acoustic signal radiated when the phase-change nano droplet ultrasonic contrast agent is caused to have a phase change and microbubbles are generated, when used for the purpose of measuring a spectrum change of the acoustic signal, it is desirable that the means be configured to be capable of measuring an ultrasound in a frequency bandwidth same as and from approximately one-tenth to ten-times of that of the ultrasound used for the phase change of the phase-change nano droplet ultrasound contrast agent at intervals of approximately 1 µm or smaller.

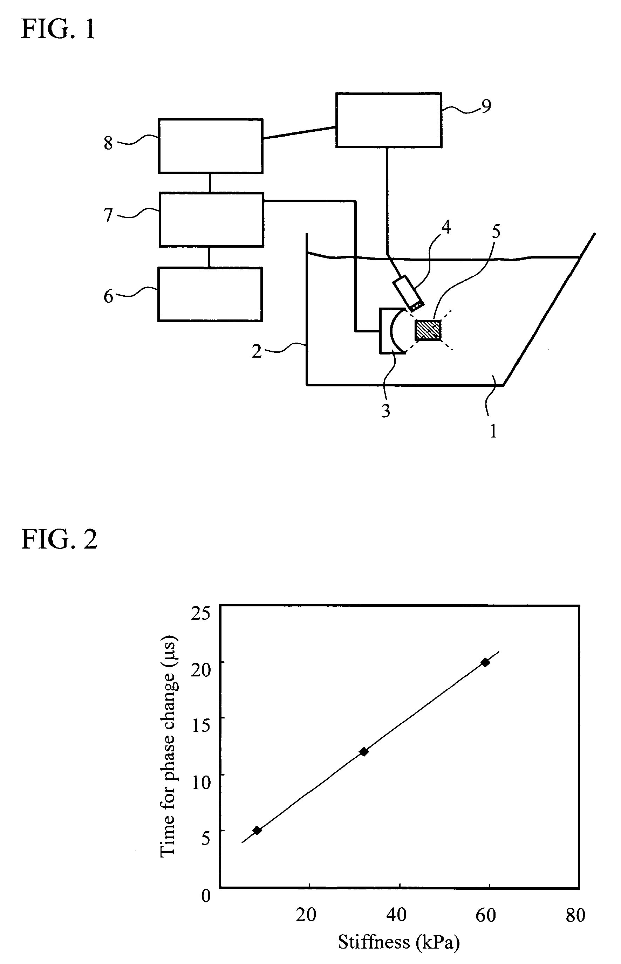

Hereinafter, an example of a test for demonstrating the effectiveness of the present invention will be described. Fig. 1 illustrates a configuration of an experiment system for the test. In a water tank 2 filled with degassed purified water 1, a focused ultrasound transducer 3 (frequency: 3.2 MHz, diameter: 60 mm, F value: 1.2) held by a holder not shown in the diagram is arranged as well as an underwater microphone 4 held by a holder not shown in the diagram and located at a position where an acoustic signal from a focus region of the focused ultrasound transducer 3 can be detected. In the focus region of the focused ultrasound transducer 3, a polyacrylamide gel 5 containing phase-change nano droplets which are prepared in a method described below is fixed with a holder not shown in the diagram.

### Preparation of phase-change nano droplets

The following components were mixed together at once to obtain a mixture. Thereafter, the mixture was gradually added with 20 ml of distilled water while being homogenized at 9,500 rpm in ULTRA TURRAX T25 (Janke & Knukel, Staufen, Germany) at ice temperature for 1 minute:

| | |
|---|---|
| Glycerol | 2.0 g |
| Cholesterol | 0.1 g |
| Lecithin | 1.0 g |
| Perfluoropentane | 0.1 g |
| Perfluoroheptan | 0.1 g |

An emulsion thus obtained in the homogenization process described above was subjected to high-pressure emulsification treatment at 20 MPa in Emulsiflex-C5 (Avestin Inc., Ottawa, Canada) for 2 minutes, and then to filtration using a membrane filter having a size of 0.4 microns. After this process, virtually transparent nano droplets were obtained. It was measured and confirmed by using LB-550 (HORIBA, Ltd., Kyoto, Japan) that at least 98% of the obtained nano particles had a diameter of 200 nm or smaller.

### Preparation of a polyacrylamide gel containing phase-change nano droplets

One mill-liters of the nano particle droplets obtained in the above-described preparation method was added and mixed with 40% acrylamide stock solution, which is prepared by adding distilled water to a mixture of 390 g of acrylamide and 10 g of N, N'-methylene-bis-acrylamide to bring the final volume of 1,000 ml, and distilled water to prepare 9 ml of dispersion liquids having an acrylamide concentration of 8%, 15%, and 20%, respectively. Each of the mixture liquids was transferred to a container having an inside dimension of 65 cm × 15 cm × 10 cm, held at ice temperature, added with 0.1 ml of 10% ammonium persulfate (APS) solution and 0.01 ml of N,N,N',N'-Tetramethylethylenediamine (TEMED), and then immediately but gently stirred so as not to generate bubbles. Thereafter, the mixture liquids were left at ice temperature for 10 minutes. After having been confirmed to have turned into a gel, the individual mixture liquids were rinsed with distilled water to obtain a virtually transparent polyacrylamide gel containing the phase-change nano droplets. The stiffness of these polyacrylamide gels was measured by a method in which a degree of bending of a gel having both ends thereof being supported is measured.

While the gel containing phase-change nano droplet 5 prepared in the above-described method and fixed in the water tank 2 was held in a focus region of the focused ultrasound transducer 3, a sine wave having a frequency of 3.2 MHz was generated in a waveform generator 6, amplified in an amplifier 7, and then entered to a transducer 3 so as to irradiate the gel containing phase-change nano droplet 5 with an ultrasound having a peak negative pressure of 3 MPa for 100 µs. An electric signal waveform during the irradiation and an acoustic signal detected by the underwater microphone 4 were measured by an oscilloscope 8 via a preamplifier 9. It should be noted that it is configured that an electric signal sent to the focused ultrasound transducer 3 triggers the initiation of the measurement of a signal obtained in the preamplifier 9 by amplifying an acoustic signal which has been detected by the underwater microphone 4.

Hereinafter, a description will be given of an example of a result obtained in the experiment system illustrated in Fig. 1. Fig. 2 is a diagram illustrating an example of the relationship between the stiffness of the gel and a time from the initiation of ultrasound irradiation set as time zero to the time point when an acoustic signal, for example, a second harmonic component of the irradiated ultrasound, from microbubbles generated due to a phase change of the nano droplets, becomes measurable. The diagram indicates that a time required for causing the phase change in the gel is longer when the stiffness of the gel is higher, and that the time increases approximately linearly in respect to the stiffness of the gel. It is also observed that the time required for causing the phase change in the gel is in an approximate range from 5 µs to 25 µs in the range of stiffness shown in Fig. 2.

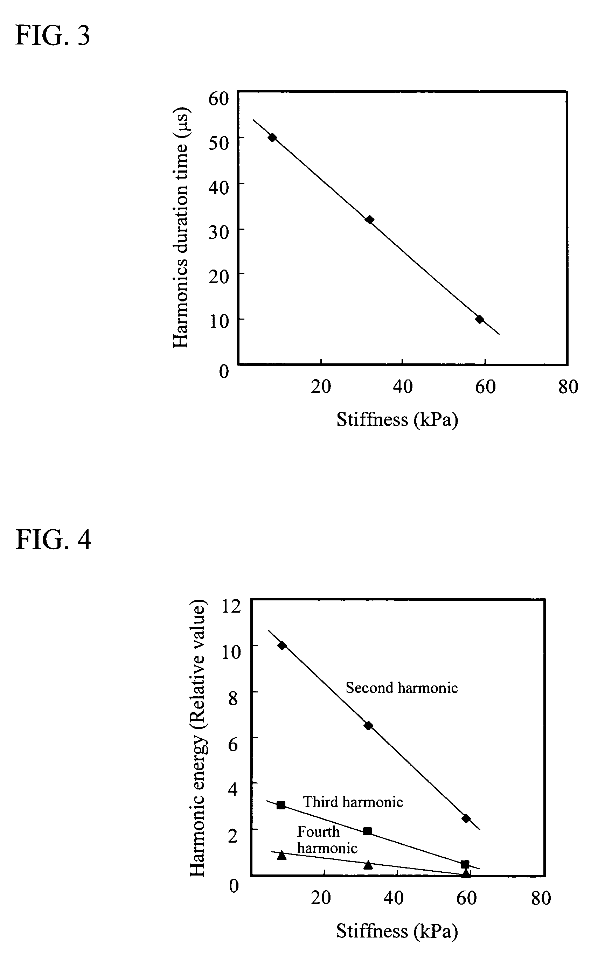

Fig. 3 is a diagram illustrating an example of the relationship between the stiffness of the gel and a time of the radiation of a second component having a frequency of 6.4 MHz from microbubbles generated due to a phase change of the nano droplets. The diagram indicates that the duration time of the radiation of a second harmonic component generated due to a phase change is shorter when the stiffness of the gel is higher, and that the duration time is shortened approximately linearly in respect to the stiffness of the gel. It is also observed that the duration time of the second harmonic component generated due to a phase change and maintained is approximately 10 µs to 50 µs.

Fig. 4 is a diagram illustrating an example of the relationships between the stiffness of the gel and the energies of respective harmonic components (second, third, and fourth harmonic components) in the microbubbles generated due to a phase change of the nano droplets. A radiant energy was calculated by adding squared amplitudes of the respective harmonic components at individual measurement time points. The diagram indicates that the energies of the respective harmonic components generated due to the phase change are smaller when the stiffness of the gel is higher, and that the energies decreases approximately linearly in respect to the stiffness of the gel.

The above-described results revealed that the time required for causing the phase change of the phase-change nano droplets, the duration times of the respective harmonic components due to the phase change, and the radiant energies of the respective harmonic components each change dependently on the stiffness of a part where the phase change occurs. Hence, by using this principle, it is possible to measure tissue characteristics.

The present invention is capable of measuring and displaying tissue characteristics when used in combination with a phase-change nano droplet ultrasonic contrast agent, and also allows a therapy to be integrated to the present invention; thus, safe and reliable diagnosis and therapy can be provided.

### BRIEF DESCRIPTION OF THE DRAWINGS

Fig. 1 is a diagram illustrating configuration of an experiment system for a test example of the present invention.
Fig. 2 is a diagram illustrating the relationship between the stiffness of a sample and a time for phase change, obtained in the test example of the present invention.
Fig. 3 is a diagram illustrating the relationship between the stiffness of a sample and a duration time of the generation of a harmonic component due to a phase change.
Fig. 4 is a diagram illustrating the relationships between the stiffness of a sample and relative sizes of the respective harmonic energies due to a phase change.
Fig. 5 is a diagram illustrating an embodiment of a tissue function measurement device of the present invention.
Figs. 6A to 6C are diagrams illustrating an example of a functional image superimposed on a conventional image in an embodiment of the present invention.
Figs. 7A to 7C are diagrams illustrating an example of a functional image superimposed on a conventional image in an embodiment of the present invention.
Fig. 8A to 8C is a diagram illustrating an example of a functional image superimposed on a conventional image in an embodiment of the present invention.
Fig. 9 is a diagram illustrating an embodiment of a tissue function measurement device of the present invention.
Fig. 10 is a diagram illustrating an embodiment of a tissue function measurement device of the present invention.
Fig. 11 is a diagram illustrating an embodiment of a tissue function measurement and therapeutic device of the present invention.

### DESCRIPTION OF THE PREFERRED EMBODIMENTS

Hereinafter, embodiments of the present invention will be specifically described. However, the present invention is not limited to these embodiments.

### [Embodiment 1]

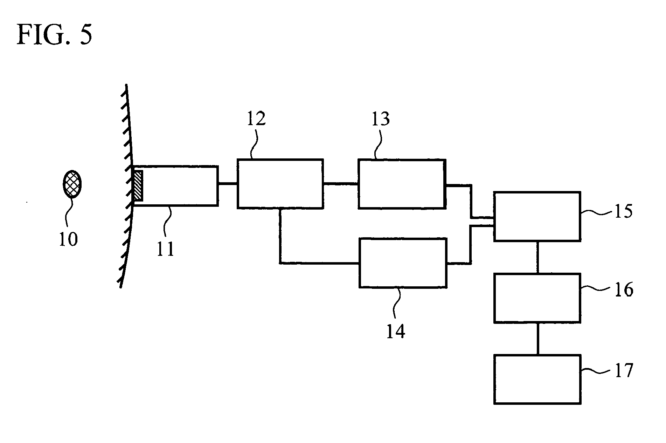

A first embodiment of the present invention will be described by referring to Fig. 5. Fig. 5 is a schematic diagram illustrating an example of an ultrasound imaging system in which a means for generating an ultrasound for causing a phase change is also serving as a means for measuring tissue characteristics.

An ultrasound imaging system of the present embodiment has: a conventional imaging mode for acquiring an ultrasound tomographic image of a measurement target by irradiating the measurement target with a tomographic imaging ultrasound and then receiving an ultrasound signal reflected from the inside of the tissue; and a tissue functional imaging mode for acquiring a tissue functional image of the measurement target by irradiating a phase-change nano droplet ultrasound contrast agent incorporated into the measurement target in advance with a phase-changing ultrasound capable of causing a phase change of the phase-change nano droplet ultrasound contrast agent to generate microbubbles, and then receiving an ultrasound signal derived from the generated microbubbles. The ultrasound imaging system includes: an ultrasound transmitting and receiving section 11; a transmitted- and received-wave separating section 12; an ultrasound waveform generating and amplifying section 13; a tissue function-dependent signal recording section 14; a transmitted- and received-wave integration control section 15; a tissue function-dependent signal processing section 16; and an input and display section 17. The ultrasound transmitting and receiving section 11 is configured to irradiate a measurement target 10 with a tomographic imaging ultrasound and receive a signal reflected from the measurement target 10, or to irradiate the measurement target 10 with a phase-changing ultrasound and receive a tissue function-dependent signal generated from microbubbles.

It should be noted that this system is also used for signal transmission and reception, signal processing, and image display of a conventional ultrasound tomographic image. As for a conventional ultrasound tomographic image, a diagnostic ultrasound signal, which has been generated and amplified in the amplifying section 13, is sent to the measurement target 10 by the ultrasound transmitting and receiving section 11, and then received by the ultrasound transmitting and receiving section 11, sent via the transmitted- and received-wave separating section 12, processed in an A/D converter, a phasing section, and a processing section for diagnostic signal, which are not shown in the diagram, and then displayed in the input and display section 17.

In the conventional imaging mode for acquiring a conventional ultrasound tomographic image, the ultrasound transmitting and receiving section 11 selects an appropriate ultrasound having a frequency in an approximate range from 1 MHz to 10 MHz according to the depth of a measurement target from the body surface thereof as well as the shape of the measurement target. Meanwhile, in the tissue functional imaging mode in which a phase-changing ultrasound is irradiated to a measurement target in order to cause a phase change of nano droplets contained in the measurement target and a tissue function-dependent signal from microbubbles generated due to the phase change of the nano droplets, the ultrasound transmitting and receiving 11 is configured to receive either an ultrasound having a single frequency selected from a frequency range from 0.5 MHz to 10 MHz or an ultrasound having a basic frequency selected from a frequency range from 0.5 MHz to 10 MHz and an ultrasound having a frequency double of the basic frequency. In the case where a time required for the generation of microbubbles from the nano droplets due to a phase change is used as a tissue function-dependent parameter, the present embodiment is configured so as to be able to adjust timings of sending and receiving wave signals in order to examine whether or not an acoustic signal from a single site varies in an approximate time range from 10 µs to 50 µs. In addition, in the case where the energy of the harmonic component is used as a tissue function-dependent parameter, the present embodiment is configured so as to be able to record and perform the four-function calculation on an amplitude or a squared amplitude of a received signal for each frequency. The tissue function-dependent signal processing section 16 is capable of performing a processing on a tissue function-dependent parameter so that the parameter can be expressed as a numerical value or an image, and further displayed as being superimposed on a diagnostic image (ultrasound tomographic image) at a certain superimposition rate.

Hereinafter, a description will be give of an example of a procedure for image display in the present embodiment. Firstly, the phase-change nano droplet contrast agent is administered to a subject who needs a diagnosis or a diagnostic treatment by a medically-endorsed method, such as intravenous injection and local injection. After a predetermined time, first, the system is set to the conventional imaging mode, and then the ultrasound transmitting and receiving section 11 irradiates a measurement target 10 with an ultrasound and then receives a reflected ultrasound thereof so as to acquire an ultrasound tomographic image of the measurement target 10. Next, the system is set to the tissue functional imaging mode, and the ultrasound transmitting and receiving section 11 sends a phase-changing ultrasound signal to the measurement target 10 for 5 µs. Thereafter, the ultrasound transmitting and receiving section 11 receives a signal from the measurement target 10 at intervals of 0.03 µs for 50 µs. The signal thus received is recorded in the tissue function-dependent signal recording section 14, and then processed in the tissue function-dependent signal processing section 16 so that a second harmonic component of the received signal can be extracted. Furthermore, a chronological change in the signal intensity of the second harmonic component after the irradiation of the phase-changing ultrasonic signal is recorded in the tissue function-dependent signal processing section 16. The information of the chronological change is used for calculation of tissue characteristics. For example, a time is calculated in which the signal intensity of the second harmonic at the respective sites in the measurement target is at least one-tenth of the peak value during the measurement period of 50 µs, and the length of the time is expressed as a numerical value or an image, and further displayed as being superimposed on the conventional tissue tomographic image obtained in the conventional imaging mode. In this case, since the duration time of the second harmonic is shorter when the stiffness of the tissue is higher, a region having a shorter duration time is displayed in greater emphasis. It should be noted that imaging in the conventional imaging mode may be performed after imaging is performed in the tissue functional imaging mode.

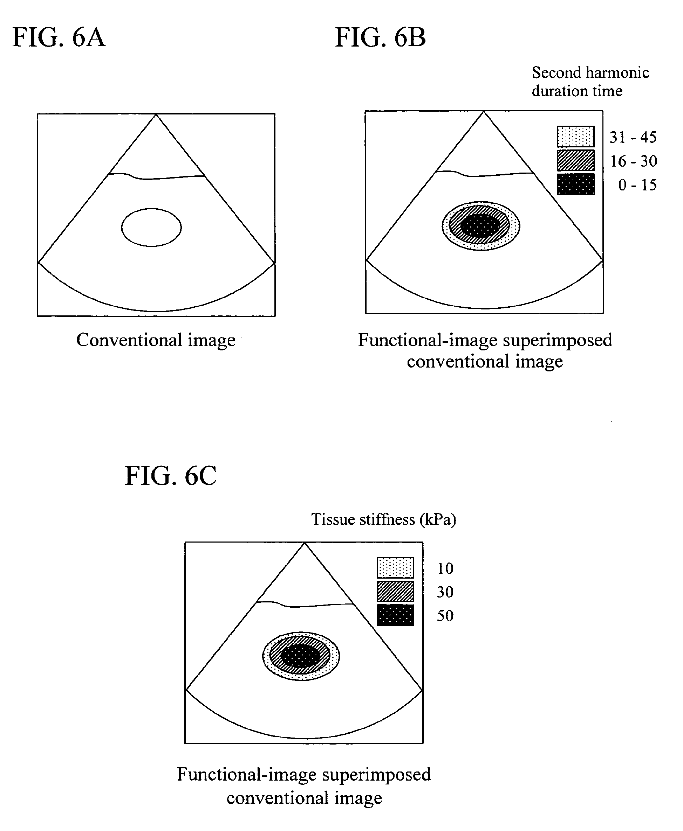

An example of displaying images is shown in Fig. 6. For superimposition of a tissue functional image, either a functional image directly showing the duration time of the second harmonic signal as illustrated in Fig. 6B, or a functional image showing the tissue stiffness calculated based on the duration time as illustrated in Fig. 6C can be used. By superimposing a conventional image and a functional image, it is possible to provide a method for confirming a disease site from both a structural standpoint and a functional standpoint. Hence, images for diagnosis and therapy having a higher accuracy can be provided to an operator. A displacement between a conventional image and a functional image on the display can be prevented by using the ultrasound transmitting and receiving section 11 for taking measurements on both the conventional image and the functional image alternatively at time intervals of approximately shorter than 1 second. In the case where a measurement target is located in a part, such as in the vicinity of the heart, significantly affected by body movement, a conventional image (image 1) is formed on the basis of a basic wave component extracted from a signal used for acquisition of a functional image, and a difference between the image 1 and a conventional image (image 2) to be used for the superimposition is measured so that a display of a functional image may be modified according to this difference. In the display illustrated in Fig. 6B, the structure and the characteristics (a time required for the generation of bubbles) of the tissue can be observed at the same time; therefore, it is possible to confirm whether or not a site having relatively different characteristics from other parts is located in a target tissue. In addition, according to the display illustrated in Fig. 6C, it is possible to confirm whether or not a site having a quantitatively different tissue stiffness is located in a target tissue.

The ultrasound transmitting and receiving section 11 allows a time of transmitting a phase-changing ultrasound signal to a measurement target 10 to be altered in accordance with purposes. A time in an approximate range from 0.5 µs to 50 ms is preferable for causing a phase change reversibly. In the meantime, the ultrasound transmitting and receiving section 11 allows a time and measurement intervals for receiving a signal from a measurement target 10 to be altered within a range below approximately 100 ms, in which the generation of harmonic due to a phase change is terminated, in accordance with purposes. While Figs. 6A to 6C are diagrams visualizing the duration time, shown in Fig. 3, of the harmonic generated from microbubbles due to a phase change, a time required for a phase change can also be visualized on the basis of the data as shown in Fig. 2. It is also possible to display a distribution of tissue stiffness by visualizing the size of the harmonic energy on the basis of the data as shown in Fig. 4.

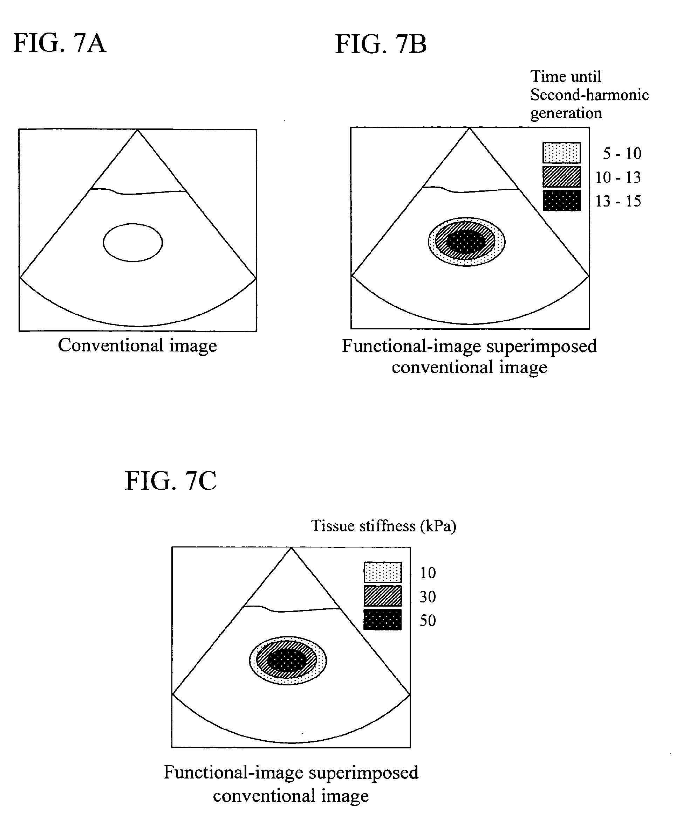

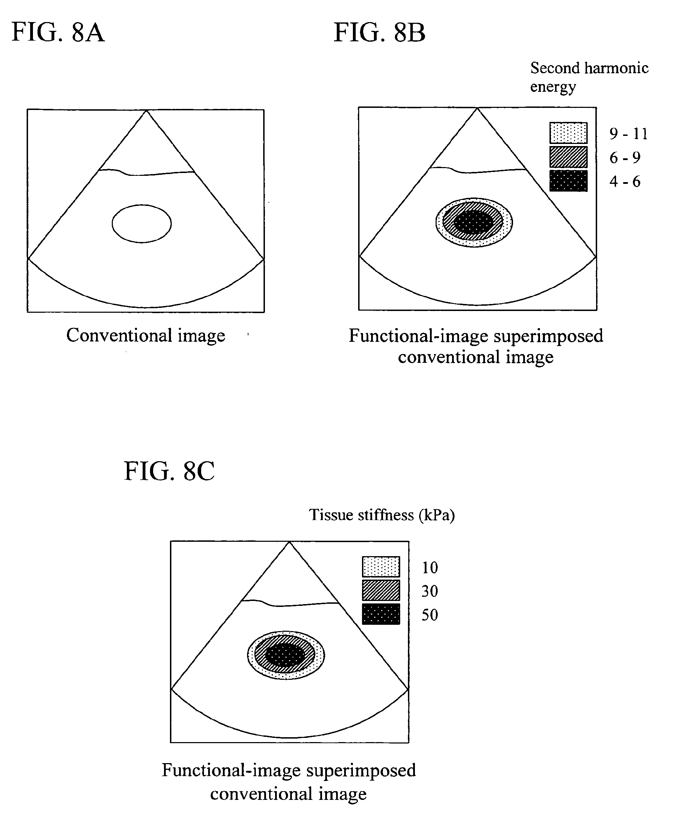

In the case of visualizing a time required for a phase change as shown in Fig. 2, the ultrasound transmitting and receiving section 11 acquires a signal at intervals in an approximate range from 0.5 µs to 5 µs up to a time of approximately 30 µs, and a time is calculated in which the amplitude of the second harmonic component in the respective signals is at least double of that at the initiation of the measurement. The calculated time expressed as an intact time value or a numerical value or an image of the length is displayed in superimposition on a conventional tissue tomographic image taken in the conventional imaging mode. An example of display is shown in Fig. 7. In the case where a time required for a phase change is visualized, a time required for the detection of the harmonic due to microbubbles is longer when the tissue stiffness is higher, as shown in Fig. 2; therefore, a region in which a time required for a phase change is longer is displayed in greater emphasis. In the case where the energy of the harmonic due to a phase change shown in Fig. 4 is visualized, for example, the ultrasound transmitting and receiving section 11 receives a signal at intervals in an approximate range from 0.5 µs to 5 µs up to a time of approximately 100 µs, and a maximum value of either the second harmonic component in the signal or the amplitude of the harmonic component is calculated. The maximum value thus obtained is squared or squared and then multiplied by a constant, and either the value thus obtained itself or the length of the value is expressed either as a numerical value or an image, and then displayed in superimposition on a conventional tissue tomographic image obtained in the conventional imaging mode. An example of a display showing the energy of the second harmonic is shown in Fig. 8. In the case where the size of the harmonic energy is visualized, the harmonic energy detected is smaller when the tissue stiffness is higher, as shown in Fig. 3; thus, a region having a smaller detected harmonic energy should be displayed in greater emphasis.

### [Embodiment 2]

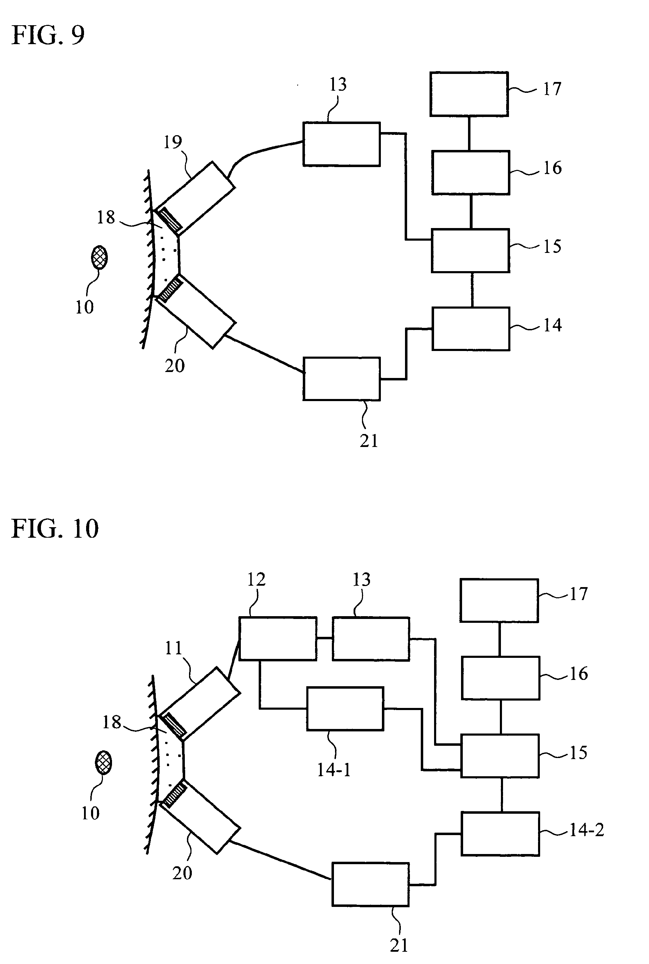

Another embodiment of the present invention will be described by referring to Fig. 9. Fig. 9 is a schematic diagram illustrating an example of a tissue function measurement device in which a means for generating an ultrasound for causing a phase change is different from a means for measuring tissue characteristics.

The ultrasound imaging system of the present embodiment has: a conventional imaging mode for acquiring an ultrasound tomographic image of a measurement target by irradiating the measurement target with a tomographic imaging ultrasound and then receiving an ultrasound signal reflected from the inside of the tissue; and a tissue functional imaging mode for acquiring a tissue functional image of the measurement target by irradiating a phase-change nano droplet ultrasound contrast agent incorporated into the measurement target in advance with a phase-changing ultrasound capable of causing a phase change of the phase-change nano droplet ultrasound contrast to generate microbubbles, and then receiving an ultrasound signal derived from the generated microbubbles. The ultrasound imaging system configured to focus on a measurement target 10 via an acoustic coupling agent 18, if necessary, includes: an ultrasound transmitting section 19; an ultrasound waveform generating and amplifying section 13; an ultrasound receiving section 20; a tissue function-dependent signal amplifying section 21; a tissue function-dependent signal recording section 14; a transmitted- and received-wave integration control section 15; a tissue function-dependent signal processing section 16; and an input and display section 17.

It should be noted that this system is also used for signal transmission and reception, signal processing, and image display of a conventional ultrasound tomographic image. As for a conventional ultrasound tomographic image, a diagnostic ultrasound signal, which has been generated and amplified in the amplifying section 13, is sent to a measurement target 10 by the ultrasound transmitting section 19, and then a reflected signal of the ultrasound signal is received by the ultrasound receiving section 20, processed in an A/D converter, a phasing section, and a processing section for diagnostic signal, which are not shown in the diagram, and then displayed in the input and display section 17.

In the conventional imaging mode for acquiring a conventional tomographic image, the ultrasound transmitting section 19 selects an appropriate ultrasound having a frequency in an approximate range from 1 MHz to 10 MHz according to the depth of a measurement target from the body surface thereof as well as the shape of the measurement target. Meanwhile, in the tissue functional imaging mode in which a phase-changing ultrasound is irradiated to a measurement target in order to cause a phase change of nano droplets contained in the measurement target and a tissue function-dependent signal from microbubbles generated due to the phase change of the nano droplets, the ultrasound transmitting section 19 is configured to receive either an ultrasound having a single frequency selected from a frequency range from 0.5 MHz to 10 MHz or an ultrasound having a basic frequency selected from a frequency range from 0.5 MHz to 5 MHz and an ultrasound having a frequency double of the basic frequency. The ultrasound receiving section 20 is configured so as to be able to receive an acoustic signal in a range from 0.5 MHz to 10 MHz. In the present embodiment, in the case where a time required for the generation of microbubbles from the nano droplets due to a phase change is used as a tissue function-dependent parameter, the present embodiment is configured so as to be able to adjust timings of sending and receiving wave signals in order to examine whether or not an acoustic signal from a single site varies in an approximate time range from 5 µs to 25 µs. In addition, the energy of the harmonic components may also be used as a tissue function-dependent parameter in the present embodiment. The tissue function-dependent signal processing section 16 is capable of performing a processing on a tissue function-dependent parameter so that the parameter can be expressed as a numerical value or an image, and further displayed as being superimposed on a diagnostic image at a certain superimposition rate. The ultrasound receiving section 20 may be an optical sensor capable of observing the generation of microbubbles from phase-change nano particles.

Hereinafter, a description will be give of an example of a procedure for image display in the present embodiment. Firstly, the phase-change nano droplet contrast agent is administered to a subject who needs a diagnosis or a diagnostic treatment by a medically-endorsed method, such as intravenous injection and local injection. After a predetermined time, first, the system is set to the conventional imaging mode. Thereafter, the ultrasound transmitting section 19 irradiates a measurement target 10 with an ultrasound and then the ultrasound receiving section 20 receives a reflected ultrasound of the irradiated ultrasound so as to acquire an ultrasound tomographic image of the measurement target 10. Next, the system is set to the tissue functional imaging mode, and the ultrasound transmitting section 19 sends a phase-changing ultrasound signal to the measurement target 10 for 5 µs. Thereafter, the ultrasound receiving section 20 receives a signal from the measurement target 10 at intervals of 0.03 µs for 50 µs. The signal thus received is amplified in the tissue function-dependent signal amplifying section 21, recorded in the tissue function-dependent signal recording section 14, and then processed in the tissue function-dependent signal processing section 16 so that a second harmonic component of the received signal can be extracted. Furthermore, a chronological change in the signal intensity of the second harmonic component after the irradiation of the phase-changing ultrasonic signal is recorded in the tissue function-dependent signal processing section 16. The information of the chronological change is used for calculation of tissue characteristics. For example, a time is calculated in which the signal intensity of the second harmonic at each of the sites in the measurement target is at least one-tenth of a peak value during a measurement period of 50 µs, and the length of the time is expressed as a numerical value or an image, and further displayed as being superimposed on the conventional tissue tomographic image obtained in the conventional imaging mode. It should be noted that imaging in the conventional imaging mode may be performed after imaging is performed in the tissue functional imaging mode.

An example of displaying images is shown in Fig. 6. For superimposition of a tissue functional image, either a functional image directly showing the duration time of the second harmonic signal as illustrated in Fig. 6B, or a functional image showing the tissue elasticity calculated according to the duration time as illustrated in Fig. 6C can be used. The ultrasound transmitting section 19 allows a time of transmitting a phase-changing ultrasound signal to a measurement target 10 to be altered in accordance with purposes. A time in an approximate range from 0.5 µs to 50 ms is preferable for causing a phase change reversibly. In the meantime, the ultrasound receiving section 20 allows a time and measurement intervals for receiving a signal from a measurement target 10 to be altered within a range below approximately 100 ms, in which the generation of harmonic due to a phase change is terminated, in accordance with purposes. While Figs. 6A to 6C are diagrams visualizing the duration time of the harmonic generated due to a phase change, a time required for a phase change can also be visualized on the basis of the data as shown in Fig. 2. It is also possible to display a distribution of tissue stiffness by visualizing the size of the harmonic energy on the basis of the data as shown in Fig. 4.

### [Embodiment 3]

Another embodiment of the present invention will be described by referring to Fig. 10. Fig. 10 is a schematic diagram illustrating an example of an ultrasound imaging system including multiple means for measuring tissue characteristics.

The ultrasound imaging system of the present embodiment has: a conventional imaging mode for acquiring an ultrasound tomographic image of a measurement target by irradiating the measurement target with a tomographic imaging ultrasound and then receiving an ultrasound signal reflected from the inside of the tissue; and a tissue functional imaging mode for acquiring a tissue functional image of the measurement target by irradiating a phase-change nano droplet ultrasound contrast agent incorporated into the measurement target in advance with a phase-changing ultrasound capable of causing a phase change of the phase-change nano droplet ultrasound contrast to generate microbubbles, and then receiving an ultrasound signal derived from the generated microbubbles. The ultrasound imaging system configured to focus on a measurement target 10 via an acoustic coupling agent 18, if necessary, includes: an ultrasound transmitting and receiving section 11; a transmitted- and received-wave separating section 12; an ultrasound waveform generating and amplifying section 13; a tissue function-dependent signal recording section 14-1; an ultrasound receiving section 20; a tissue function-dependent signal amplifying section 21; a tissue function-dependent signal recording section 14-2; a transmitted- and received-wave integration control section 15; a tissue function-dependent signal processing section 16; and an input and display section 17.

It should be noted that this system is also used for signal transmission and reception, signal processing, and image display of a conventional ultrasound tomographic image. As for a conventional ultrasound tomographic image, a diagnostic ultrasound signal, which has been generated and amplified in the amplifying section 13, is sent to a measurement target 10 by the ultrasound transmitting and receiving section 11, and then a reflected signal of the ultrasound signal is received by the ultrasound transmitting and receiving section 11, sent via the transmitted wave and received wave separating section 12, processed in an A/D converter, a phasing section, and a processing section for diagnostic signal, which are not shown in the diagram, and then displayed in the input and display section 17.

In the conventional imaging mode for acquiring a conventional tomographic image, the ultrasound transmitting and receiving section 11 selects an appropriate ultrasound having a frequency in an approximate range from 1 MHz to 10 MHz according to the depth of a measurement target from the body surface thereof as well as the shape of the measurement target. Meanwhile, in the tissue functional imaging mode in which a phase-changing ultrasound is irradiated to a measurement target in order to cause a phase change of nano droplets contained in the measurement target and a tissue function-dependent signal from microbubbles generated due to the phase change of the nano droplets, the ultrasound transmitting and receiving section 11 is configured to receive either an ultrasound having a single frequency selected from a frequency range from 0.5 MHz to 10 MHz or an ultrasound having a basic frequency selected from a frequency range from 0.5 MHz to 5 MHz and an ultrasound having a frequency double of the basic frequency. The ultrasound receiving section 20 is configured so as to be able to receive an acoustic signal in a range from 0.5 MHz to 10 MHz. In the present embodiment, in the case where a time required for the generation of microbubbles from the nano droplets due to a phase change is used as a tissue function-dependent parameter, the present embodiment is configured so as to be able to adjust timings of sending and receiving wave signals in order to examine whether or not an acoustic signal from a single site varies in an approximate time range from 10 µs to 50 µs. In addition, the energy of the harmonic components may also be used as a tissue function-dependent parameter in the present embodiment. The tissue function-dependent signal processing section 16 is capable of performing a processing on a tissue function-dependent parameter so that the parameter can be expressed as a numerical value or an image, and further displayed as being superimposed on a diagnostic image at a certain superimposition rate. In addition, the ultrasound receiving section 20 is configured so as to be able to receive a second or higher harmonic involved in the generation of microbubbles from phase-change nano particles.

Hereinafter, a description will be give of an example of a procedure for image display in the present embodiment. Firstly, the phase-change nano droplet contrast agent is administered to a subject who needs a diagnosis or a diagnostic treatment by a medically-endorsed method, such as intravenous injection and local injection. After a predetermined time, first, the system is set to the conventional imaging mode, and then the ultrasound transmitting and receiving section 11 irradiates a measurement target 10 with an ultrasound and then receives a reflected ultrasound thereof so as to acquire an ultrasound tomographic image of the measurement target 10. Next, the system is set to the tissue functional imaging mode, and the ultrasound transmitting and receiving section 11 sends an ultrasound signal for phase change to the measurement target 10 for 5 µs. Thereafter, the ultrasound transmitting and receiving section 11 and the ultrasound receiving section 20 receive a signal from the measurement target 10 at intervals of 0.03 µs for 50 µs. The signal thus received is recorded in the tissue function-dependent signal recording sections 14-1 and 14-2, and then processed in the tissue function-dependent signal processing section 16 so that a second harmonic component and a higher harmonic component, such as third and fourth components, can be extracted from the received signal. Furthermore, a chronological change in the signal intensity of the second harmonic component after the irradiation of the phase-changing ultrasonic signal is recorded in the tissue function-dependent signal processing section 16. The information of the chronological change is used for calculation of tissue characteristics. For example, a time is calculated in which the respective signal intensities of the second harmonic, the third harmonic, and the fourth harmonic at the respective sites in the measurement target are at least one-tenth of the peak value during a measurement period of 50 µs, and the length of the time is expressed as a numerical value or an image, and further displayed as being superimposed on the conventional tissue tomographic image obtained in the conventional imaging mode. It should be noted that imaging in the conventional imaging mode may be performed after imaging is performed in the tissue functional imaging mode.

An example of displaying images is shown in Fig. 6. For superimposition of a tissue functional image, either a functional image directly showing the duration time of the second harmonic signal as illustrated in Fig. 6B, or a functional image showing the tissue elasticity calculated according to the duration time as illustrated in Fig. 6C can be used. The phase-changing ultrasound transmitting and ultrasound transmitting and receiving section 11 allows a time of transmitting a phase-changing ultrasound signal to a measurement target 10 to be altered in accordance with purposes. A time in an approximate range from 0.5 µs to approximately 50 ms is preferable for causing a phase change reversibly. In addition, the phase-change ultrasound transmitting and ultrasound transmitting and receiving section 11 allows a time and measurement intervals for receiving a signal from a measurement target 10 to be altered within a range below approximately 100 ms, in which the generation of harmonic due to a phase change is terminated, in accordance with purposes. While Figs. 6A to 6C are diagrams visualizing the duration time of the harmonic generated due to a phase change, the intensity of the third harmonic or the fourth harmonic can also be visualized on the basis of the data as shown in Fig. 4. Since the third or higher harmonic is rarely included in a signal from tissue, it is possible to display a signal from bubbles generated from a phase change of nano droplets more selectively than in the case where only the second harmonic is utilized.

### [Embodiment 4]

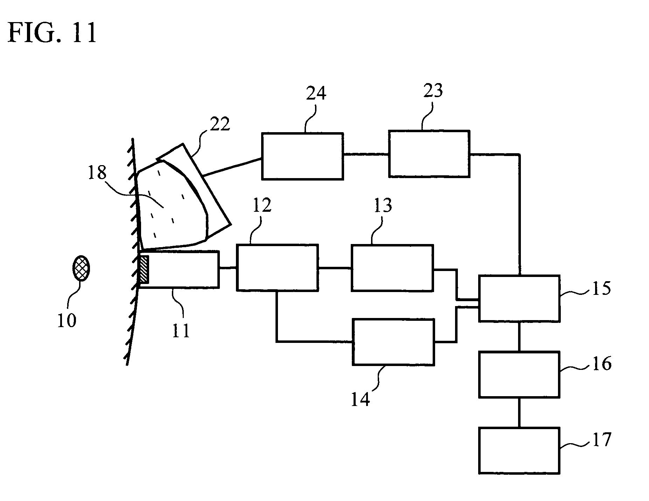

Another embodiment of the present invention will be described by referring to Fig. 11. Fig. 11 is a schematic diagram illustrating an ultrasound imaging system in which a means for generating an ultrasound causing a phase change also serves as a means for measuring tissue characteristics, and a therapeutic device utilizing microbubbles is integrated.

The ultrasound imaging system of the present embodiment has: a conventional imaging mode for acquiring an ultrasound tomographic image of a measurement target by irradiating the measurement target with a tomographic imaging ultrasound and then receiving an ultrasound signal reflected from the inside of the tissue; a tissue functional imaging mode for acquiring a tissue functional image of the measurement target by irradiating a phase-change nano droplet ultrasound contrast agent incorporated into the measurement target in advance with a phase-changing ultrasound capable of causing a phase change of the phase-change nano droplet ultrasound contrast to generate microbubbles, and then receiving an ultrasound signal derived from the generated microbubbles; and a therapeutic mode for providing therapy by irradiating a disease area with a therapeutic ultrasound.

The ultrasound imaging system configured to focus on a measurement target 10 includes: an ultrasound transmitting and receiving section 11; a separation section for transmitted and received waves 12; an ultrasound waveform generating and amplifying section 13; a tissue function-dependent signal recording section 14; a transmitted- and received-wave integration control section 15; a tissue function-dependent signal processing section 16; and an input and display section 17; an acoustic coupling media 18; a therapeutic ultrasound transducer 22; a therapeutic ultrasound transducer signal generating section 23; and a therapeutic ultrasound transducer signal amplifying section 24. The ultrasound transmitting and receiving section 11 is configured to irradiate a measurement target 10 with an ultrasound and to detect either a signal reflected from the tissue or a tissue function-dependent signal derived from microbubbles. It should be noted that this system is also used for signal transmission and reception, signal processing, and image display of a conventional ultrasound tomographic image. As for a conventional ultrasound tomographic image, a diagnostic ultrasound signal, which has been generated and amplified in the amplifying section 13, is sent to the measurement target 10 by the ultrasound transmitting and receiving section 11, and then a reflected signal of the ultrasound signal is received by the ultrasound transmitting and receiving section 11, sent via the transmitted wave and received wave separating section 12, processed in an A/D converter, a phasing section, and a processing section for diagnostic signal, which are not shown in the diagram, and then displayed in the input and display section 17.

In the conventional imaging mode for acquiring a conventional tomographic image, the ultrasound transmitting and receiving section 11 selects an appropriate ultrasound having a frequency in an approximate range from 1 MHz to 10 MHz according to the depth of the measurement target from the body surface thereof as well as the shape of the measurement target. Meanwhile, in the tissue functional imaging mode in which a phase-changing ultrasound is irradiated to a measurement target in order to cause a phase change of nano droplets contained in the measurement target and then a tissue function-dependent signal is received, the ultrasound transmitting and receiving section 11 is configured to receive either an ultrasound having a single frequency selected from a frequency range from 0.5 MHz to 10 MHz or an ultrasound having a basic frequency selected from a frequency range from 0.5 MHz to 5 MHz and an ultrasound having a frequency double of the basic frequency. The therapeutic ultrasound transducer 22 is configured differently due to a difference in therapeutic mechanism between the cases where the therapeutic mechanism is based on cavitation and heating action. To be more specific, in the case where cavitation is employed, the therapeutic ultrasound transducer 22 is configured so as to be able to irradiate a measurement target with either an ultrasound having a single frequency selected from a frequency range from 0.5 MHz to 3 MHz or an ultrasound having a basic wave frequency selected from a frequency range from 0.25 MHz to 1.5 MHz superimposed with an ultrasound having a frequency double of the basic frequency. In the case where a heating action is employed, the therapeutic ultrasound transducer 22 is configured so as to be able to irradiate a measurement target with an ultrasound having a single frequency selected from a frequency range from 1 MHz to 10 MHz.

In the present embodiment, in the case where a time required for the generation of microbubbles after the nano droplets due to a phase change is used as a tissue function-dependent parameter, it is configured such that timings of sending and receiving wave signals can be adjusted in order to examine whether or not an acoustic signal from a single site varies in an approximate time range from 5 µs to 25 µs. Meanwhile, in the case where a duration time of the generation of a harmonic component due to a phase change into bubbles is used as a tissue function-dependent parameter, it is configured such that timings of sending and receiving wave signals can be adjusted in order to examine whether or not an acoustic signal from a single site varies in an approximate time range from 10 µs to 50 µs. In addition, the energy of the harmonic components may also be used as a tissue function-dependent parameter. Furthermore, the tissue function-dependent signal processing section 16 is capable of performing a processing on a tissue function-dependent parameter so that the parameter can be expressed as a numerical value or an image, and further displayed as being superimposed on a diagnostic image at a certain superimposition rate.

Hereinafter, a description will be give of an example of a procedure for image display in the present embodiment. Firstly, the phase-change nano droplet contrast agent is administered to a subject who needs a diagnosis or a diagnostic treatment by a medically-endorsed method, such as intravenous injection and local injection. After a predetermined time, first, the system is set to the conventional imaging mode, and then an ultrasound tomographic image of a measurement target 10 is acquired. Next, the system is set to the tissue functional imaging mode, and the ultrasound transmitting and receiving section 11 sends a phase-changing ultrasound signal to the measurement target 10 for 5 µs. Thereafter, the ultrasound transmitting and receiving section 11 and the ultrasound receiving section 20 receive a signal from the measurement target 10 at intervals of 0.03 µs for 50 µs. The signal thus received is recorded in the tissue function-dependent signal recording section 14, and then processed in the tissue function-dependent signal processing section 16 so that a second harmonic component of the received signal can be extracted. Furthermore, a chronological change in the signal intensity of the second harmonic component after the irradiation of the ultrasonic signal for phase change is recorded in the tissue function-dependent signal processing section 16. The information of the chronological change is used for calculation of tissue characteristics. For example, a time is calculated in which the signal intensity of the second harmonic at the respective sites in the measurement target is at least one-tenth of the peak value during a measurement period of 50 µs, and the length of the time is expressed as a numerical value or an image, and further displayed as being superimposed on the conventional tissue tomographic image obtained in the conventional imaging mode.

An example of displaying images is shown in Fig. 6. For superimposition of a tissue functional image, either a functional image directly showing the duration time of the second harmonic signal as illustrated in Fig. 6B, or a functional image showing the tissue elasticity calculated according to the duration time as illustrated in Fig. 6C can be used. The ultrasound transmitting and receiving section 11 allows a time of transmitting a phase-changing ultrasound signal to a measurement target 10 to be altered in accordance with purposes. A time in an approximate range from 0.5 µs to 50 ms is preferable for causing a phase change reversibly. In addition, the ultrasound transmitting and receiving section 11 allows a time and measurement intervals for receiving a signal from a measurement target 10 to be altered within a range below approximately 100 ms, in which the generation of harmonic due to a phase change is terminated, in accordance with purposes. While Figs. 6A to 6C are diagrams visualizing the duration time of the harmonic generated due to a phase change, a time required for phase change can also be visualized on the basis of the data as shown in Fig. 2. It is also possible to display a distribution of tissue stiffness by visualizing the size of the harmonic energy on the basis of the data as shown in Fig. 4. The therapeutic ultrasound transducer 22 irradiates a tissue which has been confirmed to have a higher stiffness than other surrounding tissues with a therapeutic ultrasound. In this system, it may be configured that a warning sign is displayed and the irradiation with the therapeutic ultrasound is terminated if a focus region of the therapeutic ultrasound transducer 22 is set on the basis of a tissue function-dependent parameter analysis in a part which has a long phase change duration time but is not a part to be treated.

As described above, according to the present invention, it is possible to measure tissue characteristics regardless of tissue shape, to clearly distinguish between a part to be treated and a normal part, and therefore to perform safe and reliable diagnosis and therapy.

### [Description of Reference Numerals]

- 1: Degassed water
- 2: Water tank
- 3: Focused ultrasound transducer
- 4: Underwater microphone
- 5: Sample
- 6: Waveform generation device
- 7: Amplifier
- 8: Oscilloscope
- 9: Preamplifier
- 10: Measurement target
- 11: Ultrasound transmitting and receiving section
- 12: Transmitted and received waves separating section
- 13: Ultrasound waveform generating and amplifying section
- 14: Tissue function-dependent signal recording section
- 15: Transmitted wave and received wave integration control section
- 16: Tissue function-dependent signal processing section
- 17: Input and display section
- 18: Acoustic coupling agent
- 19: Ultrasound transmitting section
- 20: Ultrasound receiving section
- 21: Tissue function-dependent signal amplifying section
- 22: Therapeutic ultrasound transducer
- 23: Therapeutic ultrasound transducer signal generating section
- 24: Therapeutic ultrasound transducer signal amplifying section

## Claims

1. An ultrasound imaging system, comprising:
an ultrasound irradiating section for irradiating, with a ultrasound for phase change, a sample in which a phase-change nano droplet ultrasound contrast agent is incorporated;
an ultrasound receiving section for receiving an ultrasound emitted from the sample;
a signal processing section for processing a reception signal received by the ultrasound receiving section and detecting a harmonic component of the ultrasound irradiated from the ultrasound irradiation section; and
a display section for displaying an image based on a detection result of the harmonic component detected by the signal processing section.

2. The ultrasound imaging system according to claim 1, further comprising:
a function for acquiring an ultrasound tomographic image on the basis of a signal derived from an ultrasound for tomographic imaging irradiated from the ultrasound irradiating section and received by the ultrasound receiving section, wherein
the image based on the detection result of the harmonic component is displayed in superimposition on the ultrasound tomographic image.

3. The ultrasound imaging system according to claim 1, wherein
a time required for detection of the harmonic component from each of regions which have been irradiated with an ultrasound from the ultrasound irradiating section is determined, and
the time required for detection of the harmonic component from each of the regions is displayed as an image.

4. The ultrasound imaging system according to claim 3, wherein a region in which the time is longer is displayed in greater emphasis.

5. The ultrasound imaging system according to claim 1, wherein
a duration time of a harmonic component detected from each of regions which have been irradiated with an ultrasound from the ultrasound irradiating section is determined, and
the length of the duration time is displayed as an image.

6. The ultrasound imaging system according to claim 5, wherein a region in which the duration time is shorter is displayed in greater emphasis.

7. The ultrasound imaging system according to claim 1, wherein
the size of an energy of a harmonic component detected from each of regions which have been irradiated with an ultrasound from the ultrasound irradiating section is determined, and
the size of the energy is displayed as an image.

8. The ultrasound imaging system according to claim 7, wherein a region in which the energy is smaller is displayed in greater emphasis.

9. The ultrasound imaging system according to claim 1, wherein the display section displays the stiffness of a region irradiated with an ultrasound from the ultrasound irradiating section, on the basis of the result of detection of the harmonic component.

10. The ultrasound imaging system according to claim 1, further comprising:
a second ultrasound irradiating section for irradiating a therapeutic ultrasound, wherein
the second ultrasound irradiating section irradiates, with a therapeutic ultrasound, any one of: a region in which the time required for the detection of the harmonic component is longer than a predetermined value; a region in which the duration time of the detected harmonic component is shorter than a predetermined value; and a region in which the size of an energy of the detected harmonic component is smaller than a predetermined value.

11. The ultrasound imaging system according to claim 1, wherein the ultrasound irradiating section also serves as the ultrasound receiving section.

12. The ultrasound imaging system according to claim 1, wherein the ultrasound receiving section includes: a first receiving part for receiving a first harmonic component of an ultrasound irradiated from the ultrasound irradiating section; and a second receiving part for receiving a harmonic component of the ultrasound having a higher frequency than the first harmonic component.
